Europäisches Patentamt

European Patent Office

Office européen des brevets

(11) Publication number: **0 293 227**
**A2**

(12) # EUROPEAN PATENT APPLICATION

(21) Application number: 88304835.7

(22) Date of filing: 27.05.88

(51) Int. Cl.⁴: **F 16 M 11/12**
G 02 B 7/00

(30) Priority: 29.05.87 JP 134504/87
30.06.87 JP 161249/87

(43) Date of publication of application:
30.11.88 Bulletin 88/48

(84) Designated Contracting States: CH DE GB IT LI

(71) Applicant: **Mitaka Kohki Co., Limited**
5-1-4, Ohsawa
Mitaka-shi Tokyo (JP)

(72) Inventor: **Nakamura, Katushige**
653-1, Tatemachi
Hachioji-shi Tokyo (JP)

(74) Representative: **Gold, Tibor Zoltán et al**
T.Z.GOLD & COMPANY 9, Staple Inn
London WC1V 7QH (GB)

(54) Stand mechanism for a medical optical equipment.

(57) Since an optical equipment is supported by a parallel linkage and the balance of weight with respect to all directions such as vertical, horizontal and turning directions of the parallel linkage is maintained, the optical equipment looks as if it would be floating in the air and thereby can be moved to any desired position with small strength, and hence the operation can be carried out more exactly and more rapidly.

FIG.1

EP 0 293 227 A2

## Description

### Stand Mechanism for a Medical Optical Equipment

#### Background of the Invention

The present invention relates to a stand mechanism for supporting a medical optical equipment.

An encephalic surgical operation and a cardiac surgical operation are implemented by observing the seat of a disease with a medical optical equipment, i.e., a surgical microscope, and such very delicate operations impose a high strain on the nerves and, in most cases, requires a long time, which undesirably causes both the operators and the patient physical and spiritual fatigue.

A medical optical equipment such as a surgical microscope plays a very significant role in advanced surgical operations, and the accessibility of the medical optical equipments has direct effect on reducing time necessary for a surgical operation. The accessibility of the medical optical equipment, namely, the possibility of positioning the medical optical equipment accurately and quickly at an objective position corresponding to the seat of a disease, the possibility of moving the medical optical equipment away from the region of operation to and fixedly positioning the same at an optional standby position and the stability of the medical optical equipment at a fixed position, is dependent mostly on the performance of a stand mechanism supporting the medical optical equipment.

Various stand mechanisms for supporting a medical optical equipment including such as disclosed in Japanese Patent Provisional Publication(Kokai) No. 56-32110 have been proposed. However, those known stand mechanism are not sufficiently satisfactory in respect of accessibility in view of practical medical operations.

#### Brief Summary of the Invention

The present invention has been made in view of those disadvantages of the prior art and it is therefore an object of the present invention to provide a stand mechanism for supporting a medical optical equipment, capable of properly functioning in practical medical operations and capable of enabling the medical optical equipment to demonstrate the utmost functions thereof.

Various other features and advantages of the invention will be more apparent by referring to the following description and the accompanying drawings.

#### Brief Description of the Drawings

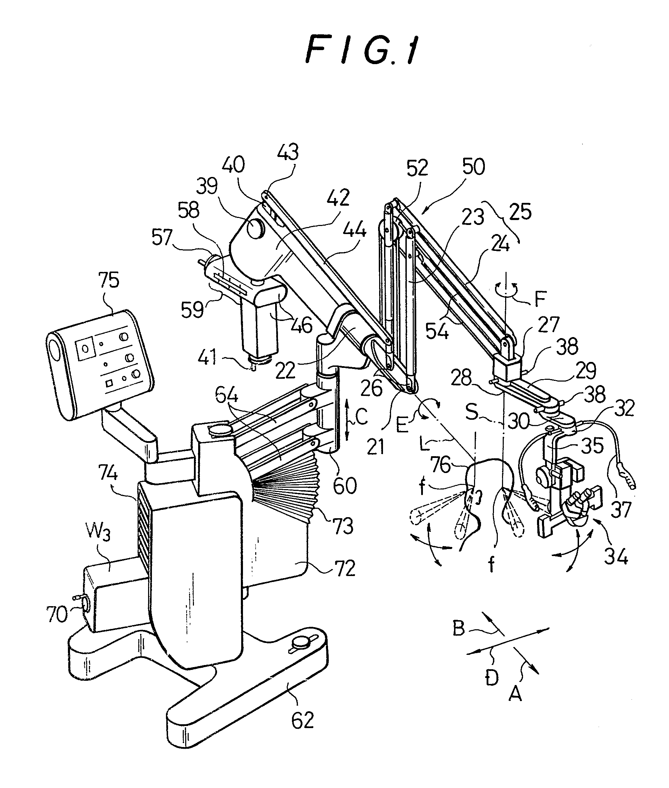

Figure 1 is a general perspective view of a stand mechanism for a medical optical equipment, embodying the present invention;

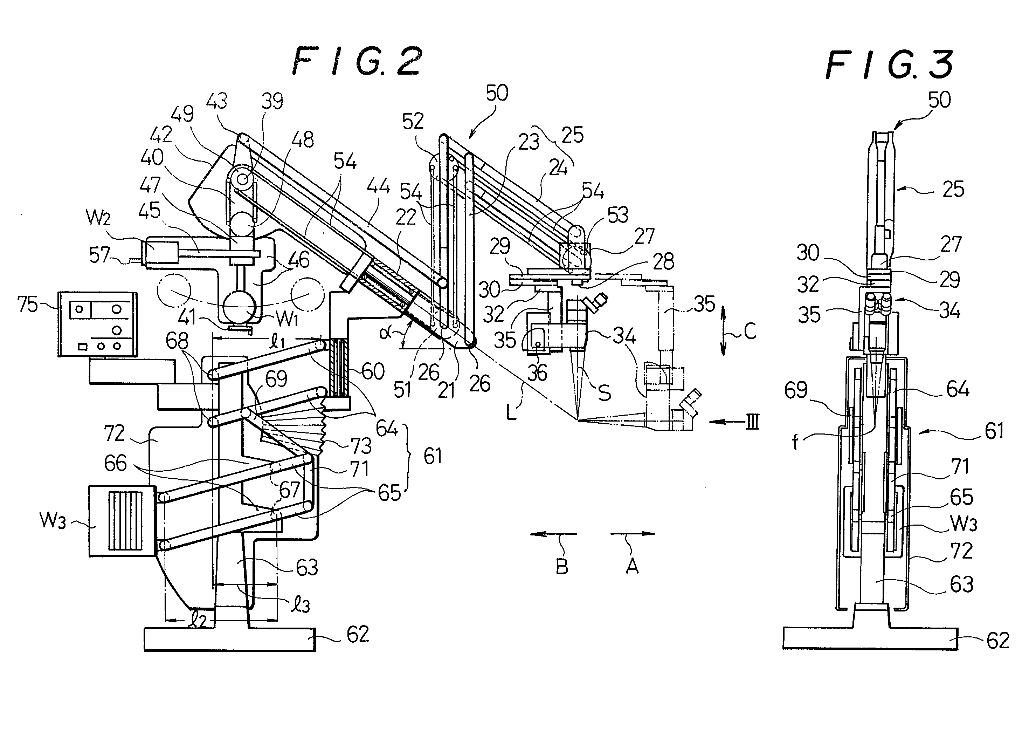

Figure 2 is a schematic side elevation showing the weight balancing system of the stand mechanism of Fig. 1;

Figure 3 is a schematic side elevation as viewed in the direction of an arrow III in Fig. 2;

Figure 4 is a diagrammatic illustration of assistance in explaining the operation of a parallel linkage in adjusting the observation angle of a medical optical equipment in a plane including the parallel linkage;

Figure 5 is a fragmentary perspective view of a mechanism for swinging a counterweight according to the turning motion of a swivel plate in a horizontal plane;

Figure 6 is a schematic plan view of assistance in explaining the motion of the stand mechanism of Fig. 1 in moving the medical optical equipment in longitudinal directions;

Figure 7 is a schematic plan view of assistance in explaining the motion of the stand mechanism of Fig. 1 in moving the medical optical equipment in lateral directions;

Figure 8 is a side elevation of the medical optical equipment held in a vertical position;

Figures 9(a) to 9(c) are side elevations showing processes of changing the position of the medical optical equipment from a vertical position to a 45°-inclined position; and

Figures 10(a) to 10(c) are side elevations showing processes of changing the position of the medical optical equipment from a vertical position to a 90°-inclined position.

#### Description of the Preferred Embodiments

A stand mechanism for supporting a medical optical equipment, in a preferred embodiment, according to the present invention will be described hereinafter with reference to the accompanying drawings.

First the constitution of the stand mechanism is described, and then the operation of the stand mechanism will be described.

A center shaft 21 is supported rotatably in bearings in an inclined holding unit 22. The front end (an end projecting in a direction indicated by an arrow A) of the center shaft 21 is machined to form a plate-shaped portion. Parallel links 23 and 24 of a parallel linkage 25 are joined pivotally at the respective base ends 26 thereof to the plate-shaped portion of the center shaft 21.

A swivel plate (swivel member) 29 as a supporting member is joined pivotally to the extremity 27 of the parallel linkage 25 for turning motion about an axis 28 of rotation in a horizontal plane. A first swivel plate 30 is joined pivotally to the free end of the swivel plate 29 with a pivot shaft 31 for turning motion on the pivot shaft 31 in a horizontal plane. A second swivel plate 32 is joined pivotally to the free end of the first swivel plate 30 with a pivot shaft 33 for turning motion in a horizontal plane. A suspending arm 35 is held by the second swivel plate 32, and a surgical microscope 34 (medical optical equipment) is held pivotally on the suspending arm 35 directly below the second swivel plate 32 so as to be turnable about an axis 36 of rotation between a vertical position, a 45°-inclined position where the surgical microscope 34 is inclined at an angle of 45° and a 90°-inclined position where the surgical microscope is inclined at an angle of 90°. The focus f of the surgical microscope 34 stays always on a vertical line S passing the axis 28 of rotation regardless of the position of the surgical microscope, which will be described in detail afterward. Moreover, since the focus f of the surgical microscope 34 is positioned on the extension L of a straight line connecting the base ends 26 of the parallel linkage 25 aligned with the extension of the center axis of center shaft 21, the position of the focus f remains unchanged when the parallel linkage is transformed. A handle 37 is provided for moving (swinging) the surgical microscope 34. Fixtures 38 are provided respectively at the base end and front end of the swivel plate 29 to change the position of the handle 37 according to the position of the operator.

A pnedulous first counterweight W1 is suspended by a swing bar 40 from the rear end (an end projecting in a direction indicated by an arrow B) of the center shaft 21 so as to be able to swing on a pivot shaft 39. An adjusting handle 41 is associated with the first counterweight W1 to adjust the vertical position of the first counterweight W1. The root portion of the swing bar 40 are covered with a cover 42. The first counterweight W1 and a second counterweight W2 are covered with another cover 46.

The upper end 43 of the swing bar 40 and the parallel linkage 25 are interconnected by a connecting bar 44 to interlock the parallel linkage 25 and the first counterweight W1. Thus, the first counterweight W1 counterbalances the inclusive weight of the surgical microscope 34 and the parallel linkage 25, and the rotatory moment of the parallel linkage 25 with respect to the center axis of the center shaft 21. Since the center shaft 21 is inclined at an angle $\alpha$ to a horizontal line, the first counterweight W1 and the associated parts are excluded from an operating zone, so that the sufficiently wide and free operating zone can be secured for the operator.

The second counterweight W2 is connected by another swing bar 45 to the middle of the swing bar 40 suspending the first counterweight W1. The second counterweight W2 is able to swing in a vertical plane to the front and rear together with first counterweight W1 and is able to be swung in a horizontal plane by a mechanism which will be described afterward. That is, the base end of the swing bar 45 for the second counterweight W2 is fixed to a pivot shaft 47 which is coaxial with the swing bar 40, and the second counterweight W2 moves along a semicircular path behind the pivot shaft 47. The pivot shaft 47 is interlocked with a rotary disk 49 provided near the rear end of the center shaft 21 through a pair of bevel gears 48 respectively having axes of rotation perpendicular to each other. Disks 51, 52 and 53, which are similar to the disk 49, are provided rotatably respectively near the base ends 26 of the parallel linkage 25, the articulated portion 50 of the parallel linkage 25 and the extremity 27 of the parallel linkage 25. One of the bevel gears 48 and the disks 49, 51, 52 and 53 are interlocked by pairs of link bars 54 to transmit a torque therethrough. The disk 53 provided near the extremity 27 of the parallel linkage 25 is interlocked with the swivel plate 29 by a pair of bevel gears 55. One of the bevel gears 55 is fixed to the disk 53 and the other bevel gear 55 is fixed to a pivot shaft 56 fixed to the swivel plate 29 and engages the former bevel gear 55. Accordingly, when the swivel plate 29 is turned in a horizontal plane, the turning motion of the swivel plate 29 is transmitted through the pivot shaft 56, the bevel gears 55, the disk 53, the disk 52, the disk 51, the disk 49 the bevel gears 48 and the pivot shaft 47 in that order to the swing bar 45 holding the second counterweight W2 to turn the swing bar 45 in a horizontal plane in a direction the same as that of turning motion of the swivel plate 29, so that the swivel plate 29 and the swing bar 45 holding the second counterweight W2 extend respectively in opposite directions. Thus, the rotatory moment with respect to the center shaft 21 produced by the turning motion of the swivel plate 29 is counterbalanced. The position of the second counterweight W2 on the swing bar 45 can also be adjusted according to the inclination (0°, 45° or 90°) of the surgical microscope 34 by turning an adjusting handle 57. The slit 58 is formed in a portion of the cover 46 covering the second counterweight W2. Three notches 59 are formed in the slit 58 at positions corresponding respectively to the three inclinations of the surgical microscope 34. The position of the second counterweight W2 on the swing bar 45 can easily and accurately be adjusted according to the inclination of the surgical microscope 34 by turning the adjusting handle 57 to bring a countermark on the second counterweight W2 into alignment with one of the notches 59 corresponding to the inclination of the surgical microscope 34.

The holding unit 22 rotatably holding the center shaft 21 has a lower cylindrical part 60 rotatably supported in bearings. The cylindrical part 60 is joined to the links of a multiple parallel linkage 61. Thus, the multiple parallel linkage 61 includes the holding unit 22 and the cylindrical part 60. The multiple parallel linkage 61 supports the inclusive weight of the center shaft 21 and all the components supported on the center shaft 21 for vertical movement. The multiple parallel linkage 61 is supported pivotally on a swivel stand shaft 63 set upright for swivel motion on a base 62. The multiple parallel linkage 61 has two pairs of upper parallel links 64 and two pairs of lower parallel links 65 disposed one over the other. The lower parallel links 65 are supported pivotally respectively on pivot shafts 67 attached respectively to the extremities of projections 66 projecting from the stand shaft 64. The upper parallel links 64 are supported pivotally above the lower parallel links 65 respectively on pivot shafts 68 provided near the upper end of the stand shaft 63. The upper parallel links 64 and the lower parallel links 65 are interlocked with each other by connecting links 69. A third counterweight W3 for counterbalancing the inclusive weight of the center shaft 21 and the components supported on the center shaft 21 (hereinafter referred to "upper structure") is joined to the rear ends of the lower parallel links 65. The position of the third counterweight W3 can be adjusted by an adjusting handle 70. If a single parallel linkage is used instead of the upper parallel linkage essentially consisting of the upper parallel links 64 having an effective length $\ell_1$ (distance between the holding unit 22 and the pivot shaft 68) and the lower parallel linkage essentially consisting of the lower parallel links 65 having an effective length $\ell_2$ (distance between the pivot shaft 68 and the third counterweight W3) to support the upper structure, the effective length of the links of the single parallel linkage is the sum of the effective lengths $\ell_1$ and $\ell_2$. However, the use of the upper parallel links 64 and the lower parallel links 65 the size of the stand mechanism by the horizontal distance $\ell_3$ between the pivot shafts 67 and the pivot shafts 68, which enables the stand mechanism to be formed in a compact construction. Furthermore, since the rearward projection of the third counterweight W3 is small, the third counterweight W3 will not interfere with the peripheral equipments, which is advantageous from the viewpoint of securing safety. Connecting links 71 are provided to reinforce the lower parallel linkage of the links 65. The stand shaft 63 and the lower parallel links 65 are covered with a cover 72. Openings in the cover 72 for the vertical movement of the upper parallel links 64 and the third counterweight W3 are covered with bellows covers 73 and 74, respectively. Indicated at 75 is a control panel. The stand mechanism has electromagnetic clutches, not shown, provided respectively at the articulated portions and are operated selectively by means of a foot switch or the like to lock or free the components selectively.

The operation of the stand mechanism will be described hereinafter.

## Longitudinal Motion (Directions A and B)

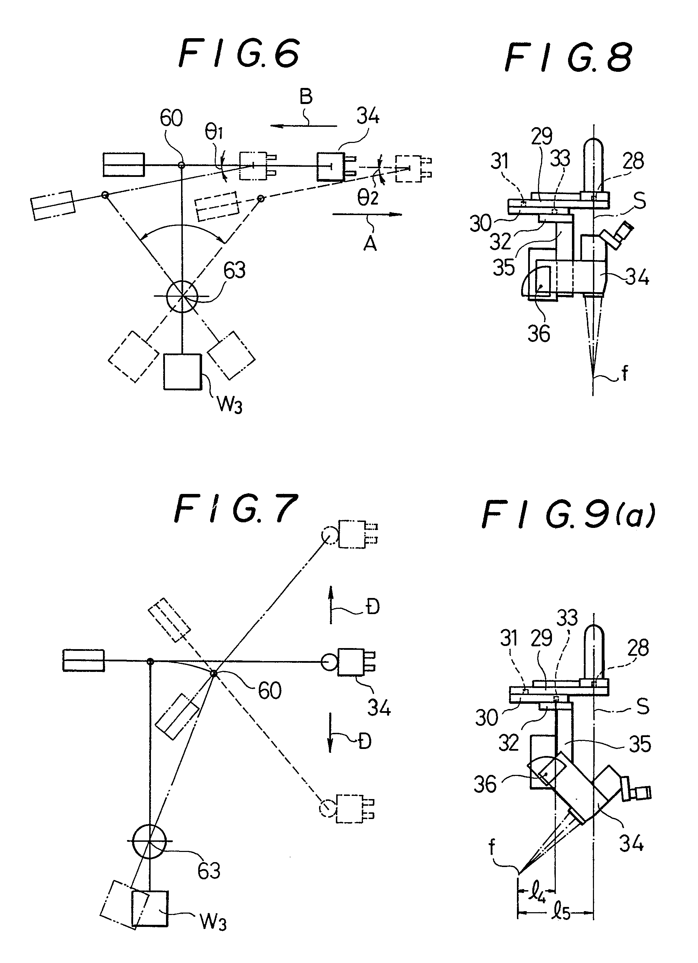

As shown in Fig. 6, when the surgical microscope is pushed in a longitudinal direction, the stand shaft 63 and the cylindrical part 60 are turned accordingly. An angle $\theta_1$ or $\theta_2$ of deviation of the surgical microscope 34 with respect to the parallel linkage 25 due to the movement of the surgical microscope 34 in the direction of the arrow B or A is compensated automatically with the turning of the swivel plate 29 holding the surgical microscope 34 in one of the directions indicated by a double-head arrow F, and hence the surgical microscope 34 can be translated in longitudinal directions without varying the observation angle.

## Vertical Motion (Directions C)

When the surgical microscope 34 is pushed in a vertical direction, the multiple parallel linkage 61 is transformed and the upper structure is moved in the same direction. Since the moment of the upper structure is counterbalanced by that of the third counterweight W3, the upper structure can easily be moved weightlessly in vertical directions.

## Lateral Motion (Directions D)

As shown in Fig. 7, when the surgical microscope 34 is pushed in a lateral direction, the stand shaft 63 and the cylindrical part 60 turn accordingly. Since the angle of deviation of the surgical microscope 34 with respect to the parallel linkage 25 attributable to the longitudinal movement of the surgical microscope 34 is compensated automatically with the turning of the swivel plate 29 holding the surgical microscope 34 in one of the directions of the double-head arrow F, the surgical microscope 34 can be translated in lateral directions without varying the observation angle.

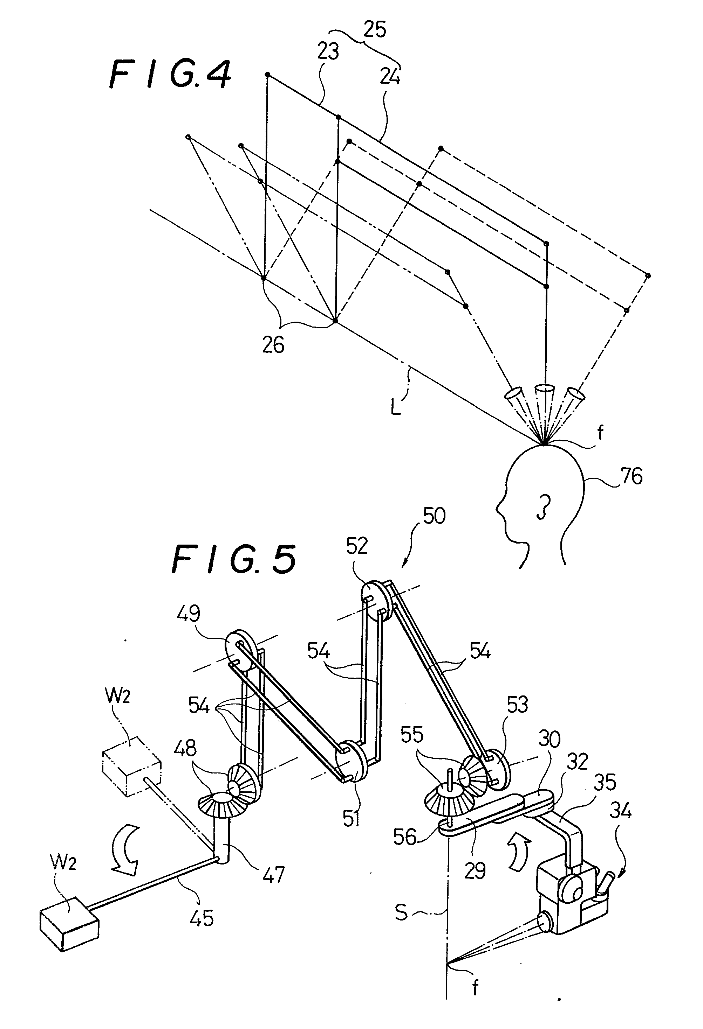

## Adjustment of Inclination of Surgical Microscope (Fig. 4)

Since the focus f of the surgical microscope 34 is on the extension L of the straight line connecting the base ends 26 of the parallel linkage 25, namely, on the extension of the center axis of the center shaft 21, the focus f remains fixed at the original position when the parallel linkage 25 is transformed. Accordingly, the observation angle can optionally be adjusted in a plane including the parallel linkage 25. When the parallel linkage 25 is transformed, both the first counterweight W1 and the second counterweight W2 are caused to swing. Therefore, the inclusive weight of the surgical microscope 34 and the parallel linkage 25 is counterbalanced by the first counterweight W1 and the second counterweight W2 to automatically maintain the balance of weight. The position of the first counterweight W1 on the swing bar 45 must be adjusted by means of the adjusting handle 41 according to the weight of accessories, such as a microscope for the assistant and a video camera, mounted additionally on the surgical microscope 34.

## Turning Motion of Center Shaft (Directions E)

When the surgical microscope 34 is turned about the center axis of the center shaft 21, the parallel linkage 25 is turned about the center axis of the center shaft 21 in the same direction, and hence the first counterweight W1 and the second counterweight W2 are turned in the same direction together with the swing bar 40. Accordingly, the balance of weight is maintained and hence the surgical microscope 34 is positioned stationary at an optional position.

## Horizontal Turning of Swivel Plate (Directions F)

Since the swivel plate 29 is capable of swivel motion in a horizontal plane, the surgical microscope 34 can be turned around the head 76 of the patient in directions of the double-head arrow F. When the swivel plate 29 is deviated from the extension of the parallel linkage 25, for example, when the swivel plate 29 is turned to a position where the swivel plate 29 extend perpendicularly to the extension of the parallel linkage 25, the swing bar 45 holding the second counterweight W2 is turned through the disks 49, 51, 52 and 53, the link bars 54 and the bevel gears 48 and 55 to a position where the swing bar 45 extends in the opposite direction. Accordingly, the balance of weight is maintained and hence the surgical microscope 34 will not turn automatically about the center axis of the center shaft 21 further in directions of the double-head arrow E.

Furthermore, since the inclination of the swing bar 40 holding the second counterweight W2 is always the same as that of the surgical microscope 34 owing to the functions of the parallel linkage 25 and the connecting arm 44, the second counterweight W2 functions perfectly in maintaining the balance of weight regardless of the position of the swivel plate 29, and hence the balance of weight with respect to the directions of turning of the center shaft 21 is never destroyed.

Still further, since the second counterweight W2 swings behind the pivot shaft 47 without varying the center of gravity of the upper structure, the counterbalancing function of the third counterweight W3 is maintained constantly.

Changing Observation Angle by Tilting Surgical Microscope

In some cases, the observation angle of the surgical microscope 34 needs to be changed through a large angle depending on the position of the operative field. For example, an encephalic surgical operation includes the operation of the parietal region of the head 76 and the operation of the temporal region of the head 76. This stand mechanism is provided with the first swivel plate 30 and the second swivel plate 32 to enable the change of the observation angle in a wide angular range.

Vertical Position:

Both the first swivel plate 30 and the second swivel plate 32 are folded below the swivel plate 29 so as to extend toward the axis 28 of rotation of the swivel plate 29 as shown in Fig. 8.

45°-Inclined Position:

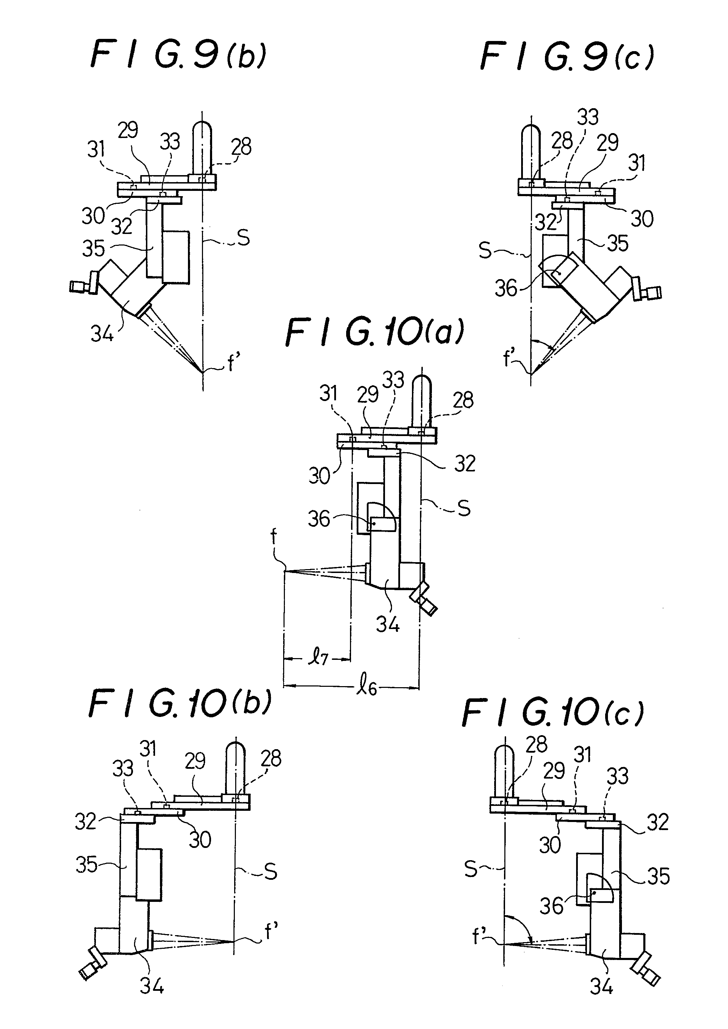

First, the surgical microscope 34 is tilted from the vertical position through an angle of 45° about the axis 36 of rotation (Fig. 9(a)). In this state, the focus f' is located on a vertical line at a distance $\ell_5$ from the vertical line S on which the focus f is located when the surgical microscope 34 is in a vertical position and at a distance $\ell_4$ from the center axis of the pivot shaft 33. The distance $\ell_5$ is exactly twice the distance $\ell_4$. Accordingly, when the second swivel plate 32 is turned through an angle of 180° on the pivot shaft 33, the focus f' is located on the vertical line S (Fig. 9(b)). Then, when the swivel plate 29 is turned through an angle of 180° about the axis 28 of rotation, the focus f' remains on the vertical line S and the operator is able to observe the operative field through the surgical microscope 34 from the same position, notwithstanding the surgical microscope 34 is tilted at an inclination of 45° (Fig. 9(c)). Accordingly, the focus f' can be brought to the original position of the focus f by slightly moving the surgical microscope 34 in a vertical direction by a lifting mechanism, not shown, incorporated into the surgical microscope 34.

90°-Inclined Position:

The surgical microscope 34 in a vertical position is turned through an angle of 90° about the axis 36 of rotation (Fig. 10(a)). In this state, the focus f' is located on a vertical line at a distance $\ell_6$ from the vertical line S and at a distance $\ell_7$ from the center axis of the pivot shaft 31. The distance $\ell_6$ is exactly twice the distance $\ell_7$. Accordingly, the focus f' can be located on the vertical line S by turning the first swivel plate 30 through an angle of 180° on the pivot shaft 31 (Fig. 10(b)). Then, when the swivel plate 29 is turned through an angle of 180° about the axis 28 of rotation, similarly to the case of 45°-inclined position, the focus f' remains on the vertical line S and the operator is able to observe the operative field through the surgical microscope 34 from the same position, notwithstanding the surgical microscope 34 is tilted at an inclination of 90° (Fig. 10(c)).

Although the multiple parallel linkage 61 of the stand mechanism in this embodiment is a two-unit parallel linkage, the multiple parallel linkage may be a three-unit parallel linkage or a multiple parallel linkage having more than three units. Furthermore, although the invention has been described as applied to supporting a surgical microscope as an exemplary medical optical equipment, the present invention is applicable to supporting various medical equipments such as a medical laser equipment. Still further, although the stand mechanism in this embodiment is a floor type stand mechanism, the present invention is applicable also to a suspension stand mechanism for the same function.

The stand mechanism for a medical optical equipment, according to the present invention provides the following effects.

(a) Since the inclusive weight of the optical equipment and the parallel linkage, and the rotary moment of the parallel linkage with respect to the center axis of the center shaft are counterbalanced by the pendulous counterweights, the stand mechanism is formed in a simple construction and is able to operate smoothly in a satisfactory balance of weight without generating noises and squeak. The employment of the rotary center shaft eliminates the need of a counterweight for balancing the weight of the parallel linkage with respect to the turning direction and simplifies the construction of the stand mechanism.

(b) Since the rotary moment with respect to the axis of the center shaft resulting from the turning motion of the supporting unit is counterbalanced by the counterweight, the balance of weight about the center shaft is maintained when the supporting unit supporting the heavy medical optical equipment is turned in a horizontal plane and the medical optical equipment can be stopped at any desired position.

(c) Since the holding unit is supported for vertical movement on the extremity of the uppermost parallel linkage of the multiple parallel linkage, and the counterweight for counterbalancing the weight of the holding unit is provided on the rear end of the lowermost parallel linkage of the multiple parallel linkage, the upper structure can easily be moved vertically. Moreover, the stand mechanism has a good appearance and, since the counterweight is not projected backward, the counterweight will not interfere with the operator and the peripheral equipments, which is advantageous from the viewpoint of safety.

(d) Since the focus of the optical equipment in the 45°-inclined position or in the 90°-inclined position can be located at a position where the focus is located when the optical equipment is in the vertical position by turning the first swivel plate or the second swivel plate through an angle of 180° in a horizontal plane, the medical optical equipment can be used at an optimum observation angle. Moreover, since the focus moves along the same vertical line, the medical optical equipment can quickly be focused remarkably reducing time necessary for a surgical operation.

## Claims

1. A stand mechanism for supporting a medical optical equipment, comprising:

a parallel linkage supporting an optical equipment on the extremity thereof;

a rotary center shaft having one end supporting the parallel linkage at the base end of the same; and

a pendulous counterweight suspended for swing motion in a vertical plane from the other end of the rotary center shaft;

characterized in that the parallel linkage and the counterweight are interlocked so that the inclusive weight of the optical equipment and the parallel linkage and the rotatory moment of the parallel linkage with respect to the center axis of the center shaft are counterbalanced by the counterweight.

2. A stand mechanism for supporting a medical optical equipment, comprising:

a parallel linkage holding a swivel member on the extremity thereof to support an optical equipment;

a rotary center shaft having one end supporting the parallel linkage at the base end of the same thereon; and

a pendulous counterweight supported for swing motion in a horizontal plane on the other end of the center shaft;

characterized in that the swivel member and the counterweight are interlocked so that a rotatory moment with respect to the center axis of the center shaft is counterbalanced by the counterweight.

3. A stand mechanism for supporting a medical optical equipment comprising:

an upper structure including a parallel linkage to support an optical equipment for movement maintaining the equilibrium of weight; and

a multiple parallel linkage comprising a plurality of parallel links disposed one over another;

characterized in that the upper structure is supported for vertical movement on one end of the uppermost link of the multiple parallel linkage, and a counterweight for counterbalancing the weight of the upper structure is provided on one end of the lowermost link of the multiple parallel linkage.

4. A stand mechanism for supporting a medical optical equipment, comprising:

an arm;

a holding unit attached to the extremity of the arm;

a first swivel plate supported for turning in a horizontal plane on the supporting unit;

a second swivel plate supported for turning in a horizontal plane about an eccentric center on the first swivel plate; and

an optical equipment suspended from the second swivel plate at the eccentric center so as to be positioned in a vertical position, an inclined position inclined at an angle of 45° with respect to a vertical direction and an inclined position inclined at an angle of 90° with respect to a vertical direction;

characterized in that when the first swivel plate or the second swivel plate is turned through an angle of 180° in a horizontal plane, the focus of the optical equipment stays always on a vertical line on which the focus of the optical equipment stays when the optical equipment is in a vertical position, regardless of the inclination of the optical equipment.

5. A stand mechanism for supporting a medical optical equipment, comprising:

a parallel linkage;

a rotary holding unit provided on the parallel linkage;

a swivel unit having first and second swivel plates capable of turning each in a horizontal plane and supporting an optical equipment and supported on the rotary holding unit;

a rotary center shaft having one end supporting the parallel linkage at the base end of the same;

a first counterweight supported on the other end of the center shaft so as to counterbalance the inclusive weight of the optical equipment and the parallel linkage and the rotatory moment of the parallel linkage with respect to the center axis of the center shaft;

a second counterweight supported on the other end of the center shaft so as to counterbalance the rotatory moment of the supporting unit with respect to the center axis of the center shaft;

a multiple parallel linkage supporting at one end thereof the center shaft for vertical movement;

a third counterweight provided on the other end of the multiple parallel linkage so as to counterbalance a weight loaded on the center shaft;

characterized in that the focus of the optical equipment in the 45°-inclined position or in the 90°-inclined position stays on the same vertical line on which the focus stays when the optical equipment is in the vertical position by turning the first swivel plate or the second swivel plate through an angle of 180° in a horizontal plane, and the focus of the optical equipment stays on the extension of the center axis of the center shaft.

FIG.1

0293227

FIG.2

FIG.3

0293227

0293227

# F I G.4

26

25
23
24

L

f

76

# F I G.5

50

52

49

54

54

54

W2

48

55

53

30

32

35

34

W2

47

51

56

29

S

45

f

0293227

# FIG.6

# FIG.8

# FIG.7

# FIG.9(a)

0293227

## F I G. 9 (b)

## F I G. 9 (c)

## F I G. 10 (a)

## F I G. 10 (b)

## F I G. 10 (c)